# EUROPEAN PATENT SPECIFICATION

(11) **EP 2 777 543 B1**
(45) Date of publication and mention of the grant of the patent: **29.11.2017**
(21) Application number: 14158741.0
(22) Date of filing: 11.03.2014
(51) Int. Cl.: A61B 17/12

(54) **Method of fabricating modifiable occlusion device**
Verfahren zur Herstellung einer modifizierbaren Okklusionsvorrichtung
Procédé de fabrication de dispositif d'occlusion modifiable

(30) Priority: 12.03.2013 US 201313796415
(43) Date of publication of application: 17.09.2014
(73) Proprietor: DePuy Synthes Products, LLC, Raynham, MA 02767 (US)
(72) Inventor: Slazas, Robert, Raynham, MA 02767 (US); Geest, Dr. Jonathan Vande, Tucson, AZ 85710 (US)
(74) Representative: Carpmaels & Ransford LLP

(56) References cited:
- US-A1- 2004 123 435
- US-A1- 2012 179 237
- US-A1- 2012 253 377

## Description

### BACKGROUND OF THE INVENTION

### 1. Field of the Invention

The invention relates to implants within body vessels and more particularly to manufacture of occlusive devices including stents which are irreversibly modified based on localized pressure differentials.

### 2. Description of the Related Art

Vascular disorders and defects such as aneurysms and other arterio-venous malformations are especially difficult to treat when located near critical tissues or where ready access to a malformation is not available. Both difficulty factors apply especially to cranial aneurysms. Due to the sensitive brain tissue surrounding cranial blood vessels and the restricted access, it is very challenging and often risky to surgically treat defects of the cranial vasculature.

In the treatment of aneurysms by endovascular methods, the goal is to exclude the internal volume of the aneurysm sac from arterial blood pressure and flow. As long as the interior walls of the aneurysm are subjected to blood pressure and/or flow, there is a risk of the aneurysm rupturing.

Non-surgical treatments include vascular occlusion devices such as embolic coils deployed using catheter delivery systems. In a currently preferred procedure to treat a cranial aneurysm, the distal end of an embolic coil delivery catheter is initially inserted into non-cranial vasculature of a patient, typically through a femoral artery in the groin, and guided to a predetermined delivery site within the cranium. The aneurysm sac is then filled with embolic material that forms a solid, thrombotic mass that protect the walls from blood pressure and flow.

One inherent drawback to embolic treatments is that the aneurysm volume is permanently maintained due to the solid embolic mass implanted within them. Even after the aneurysm walls have been relieved of blood pressure and flow impingement, the walls cannot fully heal, reshape to a less distended formation, or be reincorporated back into the parent vessel wall. Also, if the size of the aneurysm created any "mass effect" type injury to the brain, the implanted embolic mass does not allow the aneurysm to shrink significantly after treatment.

When using a neck-occlusive approach to treat an aneurysm, the entrance or "neck" of the aneurysm is treated instead of the aneurysm volume itself. If the transfer of blood across the neck can be minimized, then stasis of the blood in the aneurysm volume can lead to formation of a natural thrombotic mass without the implantation of embolic materials. A natural thrombotic mass is preferable because it allows for an increased level of healing, including reduced distension of the aneurysm walls, and perhaps possible reincorporation of the aneurysm into the original parent vessel shape along the plane of the aneurysm's neck. The neck plane is an imaginary surface where the intima of the parent artery would be if not for formation of the aneurysm.

A significant challenge for many current neck-occlusive techniques is to substantially block the aneurysm neck in the parent vessel and yet not impede flow into perforator-type vessels which branch off of the parent vessel, are very small in diameter, numerous in some anatomical locations, and yet feed clinically important regions, especially within the brain. One example is the basilar artery, which has many perforator vessels feeding the pons and upper brain stem from the parent basilar artery. The use of a non-discriminatory neck occlusive device in this type of artery can unintentionally cause severe damage to the patient if the openings, known as "ostia", of the perforator vessels are blocked.

A typical basic configuration of neck-occlusive devices is a tubular, stent-like structure. These structures can be woven or wound from various fibers, laser-cut from metal, or made in various other ways. Many have interior struts or scaffolds. What most have in common is radial symmetry, meaning that they do not cover one portion, side or radial sector of the artery more or less porously than other sectors. Their symmetric construction, and therefore coverage of artery walls, is relatively homogeneous around any given transverse slice or cross-section, except where an interior strut may further reduce porosity from a micro-level perspective.

Several embodiments of an endoluminal vascular prosthesis are described in U.S. Patent No. 6,187,036 by Shaolian et al., for example, including one embodiment having fixed perfusion ports that can be aligned with diverging arteries. This prosthesis requires careful alignment of the perfusion ports with the adjacent vessels.

One example of an occlusion device directed to sealing an aneurysm while permitting flow to adjacent vessels is disclosed in U.S. Patent No. 7,156,871 by Jones et al. An expandable stent has a covering that is normally dissolvable in blood but, upon being locally activated by an activating agent, resists dissolution where activated. This device requires precise delivery of the separate activating agent.

Another type of aneurysm occlusion system is described by Bose et al. in U.S. Patent Publication No. 2007/0239261 having a plurality of pre-formed gaps or pores which allegedly expand in response to a fluid pressure differential at a side branch vessel. Various possibilities are mentioned including deflection of bendable elements such as small paddles, elastic stretching of pores, and defeating of surface tension by increased pressure differential.

Techniques for coating stents and other medical devices include those disclosed by Hossainy in U.S. Patent No. 7,556,837, by Ruane et al. in U.S. Patent Publication No. 2008/0167724, and by Milner et al. in U.S. Patent Publication No. 2012/0179237. One technique of fabricating a highly porous tubular membrane for an arterial prosthesis is described by Soldani et al. in "Small diameter polyurethane-polydimethylsiloxane vascular prostheses made by a spraying, phase-inversion process", J. Materials Science: Materials in Medicine 3 (1992) pages 106-113.

US2004/123435 (A1) describes a machine for producing porous membranes for medical use, comprising a plurality of reserves of components which constitute fluid substances, first and second guns supplied from the reserves for spraying the fluid substances onto an element on which the substances are deposited and build up, the element and the guns being mobile relative to one another.

It is therefore desirable to manufacture a device which effectively occludes a neck of an aneurysm or other arterio-venous malformation in a parent vessel without blocking flow into perforator vessels communicating with the parent vessel.

### SUMMARY OF THE INVENTION

An object of the present invention is to optimally provide an occlusion device which substantially blocks flow into an aneurysm in a parent vessel yet quickly adapts to a pressure differential at an ostium of a perforator vessel to allow penetrating flow into the perforator vessel.

Another object of the present invention is to optimally provide an occlusion device which is sensitive to a differentiating characteristic between the neck of the aneurysm and the ostium of a perforator vessel.

This invention features a method of fabricating an occlusive device suitable for endovascular treatment of an aneurysm in a region of a parent vessel in a patient, including selecting first and second spray devices having first and second nozzle openings and first and second adjustable flow controls, respectively. A position of one unit to two units is selected for the first flow control and a position of 0.25 units to one unit is selected for the second flow control. In some embodiments, one unit is equivalent to one revolution of a flow control knob. The first and second spray devices are arranged to deliver droplets of a first liquid including at least one biocompatible polymer through the first spray device and to deliver droplets of a second liquid including a non-solvent for the polymer through the second spray device in an overlapping spray pattern on a substrate at a pre-selected distance of 25 cm to 35 cm and at a pre-selected relative translation speed of 11 cm/sec to 33 cm/sec. The at least one polymer and the non-solvent are sprayed onto the substrate to cause the biocompatible polymer to disassociate from solution to form the frangible material as a porous membrane.

In certain embodiments, the substrate is a mandrel, preferably substantially cylindrical, and the at least one polymer is biodegradable such as polycaprolactone. In some embodiments, the first liquid further includes a biocompatible polymer such as polyurethane, in a blend ratio of approximately 80:20 to 50:50.

Preferably, the frangible material initially provides a substantial barrier to flow through the frangible material and is capable of localized eroding, in the presence of a pressure differential arising at an ostium of a perforator vessel communicating with the parent vessel, within an acute time period to minimize ischemia downstream of the perforator vessel. In a number of embodiments, the method includes placing the frangible material over a structure having a fixed porosity and having dimensions suitable for insertion into vasculature of the patient to reach the region of the aneurysm in the parent vessel. In some embodiments, the structure includes metallic struts.

In certain embodiments, the frangible material includes at least one biodegradable composition. In some embodiments, the structure includes a substantially non-biodegradable porous foam, such as solidified porous urethane, and the frangible material includes at least one biodegradable composition, such as polycaprolactone, interspersed through at least a portion of the foam. In one embodiment, the frangible material is capable of responding to a pressure differential equivalent to 0.1 to 6.7 kPa (one to fifty mm Hg) and the acute time period is less than ten minutes. In some embodiments, the frangible material defines openings at least 10 microns in diameter prior to implantation in the patient and has a thickness ranging between 10 microns to 500 microns.

### BRIEF DESCRIPTION OF THE DRAWINGS AND PHOTOGRAPHS

In what follows, preferred embodiments of the invention are explained in more detail with reference to the drawings and photographs, in which:
FIG. 1 is a schematic side view of a novel occlusive device having a film fabricated according to the present invention overlying a support and positioned in a parent vessel below an aneurysm and above a perforator vessel;
FIG. 2 is a similar schematic side view of another novel occlusive device having electro-spun fibers overlying a support;
FIG. 3 is a similar schematic side view of yet another novel occlusive device having an erodible porous structure fabricated according to the present invention and covering a support;
FIG. 4A is an enlarged schematic perspective, partial cross-sectional view of a portion of an alternative embodiment to the device shown in FIG. 3 having a durable porous structure;
FIG. 4B is a view of the durable porous structure of FIG. 4A after it has been impregnated with a selectively dissolving filler material;
FIG. 5 is a schematic top view of a spray phase separation system according to the present invention;
FIG. 6 is a flow chart illustrating fabrication steps according to the present invention;
FIG. 7 is a graph of degradation of membranes formed according to the present invention with pure PCL in varying lipase concentrations; and
   PHOTOS 1-4 are scanning electron microscope images of successively smaller portions of the electro-spun fibers of the device illustrated in FIG. 2 at increasing magnifications of X15, X50, X200 and X2000, respectively.

### DETAILED DESCRIPTION OF THE PRESENTLY PREFERRED EMBODIMENTS

This invention may be accomplished by utilizing spray phase separation to fabricate an occlusive device suitable for endovascular treatment of an aneurysm in a region of a parent vessel in a patient, with at least one type of supporting structure, such as porous foam or metallic struts, and at least one type of frangible material supported by the structure. The structure has a fixed porosity and has dimensions suitable for insertion into vasculature of the patient to reach the region of the aneurysm in the parent vessel. The frangible material initially provides a substantial barrier to flow through the frangible material and is capable of at least one of localized rupturing and localized eroding, in the presence of a pressure differential arising at an ostium of a perforator vessel communicating with the parent vessel, within an acute time period to minimize ischemia downstream of the perforator vessel.

In particular, the present invention includes fabricating such an occlusive device by selecting first and second spray devices having first and second nozzle openings and first and second adjustable flow controls, respectively. A position of one unit to two units is selected for the first flow control and a position of 0.25 units to one unit is selected for the second flow control. In some embodiments, one unit is equivalent to one revolution of a flow control knob. The first and second spray devices are arranged to deliver a fine mist of droplets of a first liquid including at least one biocompatible polymer through the first spray device and to deliver a fine mist of droplets of a second liquid including a non-solvent for the polymer through the second spray device in an overlapping spray pattern on a substrate at a pre-selected distance of 25 cm to 35 cm and at a pre-selected relative translation speed of 11 cm/sec to 33 cm/sec. The at least one polymer and the non-solvent are sprayed onto the substrate to cause the biocompatible polymer to disassociate from solution to form the frangible material as a porous membrane.

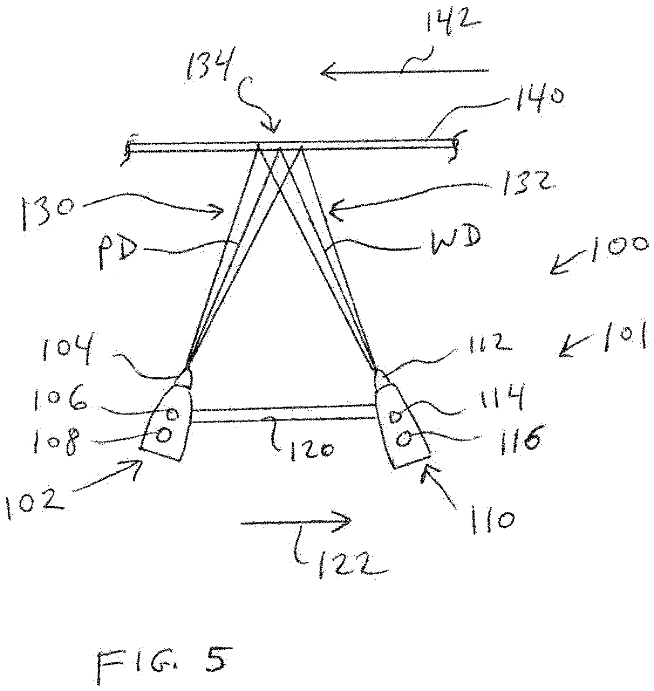

The term "spray phase separation" as utilized herein includes (1) the formation of a first droplet stream of a polymer solution and a second droplet stream of a non-solvent, and (2) intersecting the first and second droplet streams on a substrate such as a mandrel. The non-solvent causes the polymer to disassociate from solution to create a porous membrane. The term "spray phase separation" includes a "spraying, phase-inversion process" as described by Soldani et al., cited above. Spray phase separation to fabricate a suitable frangible material according to the present invention is described in more detail relating to FIGS. 5-7 below.

The parent Application No. 13//076,474 by Robert Slazas et al., US Patent Publication No. 2012/0253377, results from the realization that the neck of an aneurysm in a parent vessel can be occluded without also occluding nearby vessels, such as perforator vessels, communicating with the parent vessel by providing a device which irreversibly erodes or ruptures, including deforming, substantially only based on differential pressure and penetrating fluid flow into the perforator vessels. The device effectively senses the presence of an ostium of a perforator vessel and modifies itself to permit flow into the ostium, thereby minimizing ischemia, while continuing to substantially block flow into the aneurysm.

When considering the arterial system as a non-compressible fluid piping system, the aneurysm is a dead leg which does not drain by connecting to the low-pressure, venous side of the piping system. Over short time horizons, without considering growth or contraction of the aneurysm volume, any fluid volume that transfers across the neck plane must displace an equal amount of fluid volume from the aneurysm back into the parent vessel. The result is a net-zero transference across the neck plane for the aneurysm.

A perforator vessel differs from an aneurysm since the perforator vessel does drain directly or indirectly into the low pressure side of the piping system. There is a net-positive transference across the ostial plane because a given amount of fluid volume that crosses its ostial plane, that is, enters the perforator vessel through its ostium, is lost from the high pressure side of the system and does not force an equal amount back into the parent vessel as the aneurysm does.

In such a non-compressible fluid system, a net-zero transference across the neck plane causes a zero differential pressure across the neck plane. By comparison, a net-positive transference across the ostial plane can be detected by a positive differential pressure across the ostial plane. Therefore, differential pressure is a characteristic which a device can use to distinguish between the neck of an aneurysm and the ostia of perforator vessels. Since stent-like neck occlusion devices cover both a neck plane and an ostial plane in the same manner, the inventors of the parent application have recognized that neck occlusion devices are needed that change their flow-impeding properties according to the presence of differential pressure across their walls, from interior to exterior.

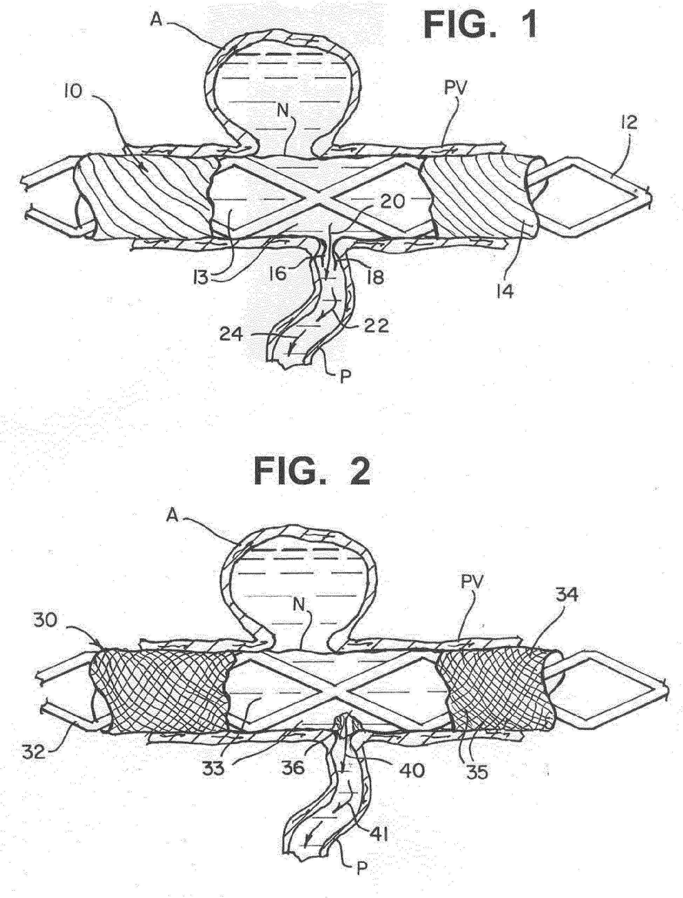

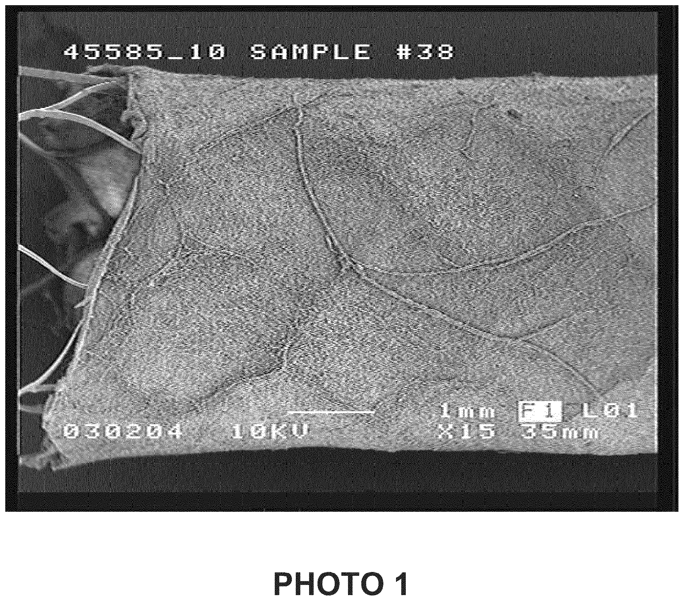

FIG. 1 schematically illustrates a tubular, stent-like device 10 fabricated according to one technique of the present invention implanted in a parent vessel PV with an upper aneurysm A and a lower perforator vessel P. Device 10 is substantially tubular and has structure such as metallic struts 12 defining relatively large openings 13 and supporting a frangible cover material 14 which includes a film-like substance that is capable of rupturing wherever a preselected differential pressure is achieved. Frangible material 14 is shown intact along the entire exterior of struts 12, including across aneurysm neck N, except where ruptured by differential pressure with resulting film flaps 16 and 18 slightly extending into the ostium of perforator vessel P. Penetrating fluid flow from parent vessel PV into perforator vessel P is illustrated by arrows 20, 22 and 24.

The frangible cover material 14 disrupts flow which would otherwise occur into aneurysm A and thereby enables a thrombus to form within aneurysm A. At the same time, frangible cover material 14 also enables blood to flow into perforator vessel P to continue feeding downstream tissues supplied by that vessel to minimize ischemia within those downstream tissues. Preferably, frangible cover material 14 provides a flow barrier at neck N for at least eight-to-twelve weeks to allow endothelial growth over device 10.

Device 10 can be either self-expanding or balloon expanded, with supporting scaffold-like structure 12 made by any of several typical stent fabrication methods. The struts 12 themselves are solid, typically metal, and do not change behavior according to the distinguishing feature of differential pressure across either an aneurysm neck or the ostium of a branching vessel. In the preferred embodiment, the struts 12 serve as a self-expanding scaffold made by laser-cutting a pattern of struts into a nitinol (NiTi) tube. The primary purposes of this structural component are to facilitate delivery of a film or other frangible cover material 14 to the target vessel, and to hold cover material 14 in apposition to the vessel wall once deployed. If the covering 14 is structurally sufficient to enable delivery and to hold position in the artery on its own, this scaffold 12 may not be needed.

The open areas 13 within the scaffold 12 are subsequently covered by a film 14 which does respond according to the level of differential pressure felt across its wall thickness. There is a net positive differential pressure across a branching vessel's ostium and none across the neck of an aneurysm, typically ranging from 0.1 to 6.7 kPa (one to fifty mm Hg). This film 14 can be made from any number of substances, as long as it has the minimum characteristics of biocompatibility and frangibility in the presence of a preselected, sufficient differential pressure. Suitable biocompatible compositions for frangible material 14 include films or matrices of cellulose, alginate, cross-linked gels, and very thin polymer films of materials such as urethane and/or poly-glycolic acid. One technique according to the present invention for fabricating the film 14 is described below relating to FIG. 5. In other constructions, the film 14 need not be erodible or bioabsorbable since it is the action of rupture in the presence of sufficient differential pressure that creates the permanent, localized modification of increased flow across its wall-thickness. Similarly, although microscopic pores or other openings could be formed in the film 14 having average diameters such as described for other embodiments below, it is acceptable for the film 14 to be a continuous sheet of material because the action of rupture increases flow where needed, as sensed by sufficient differential pressure to cause the rupture.

The thickness of the film layer is determined by its desired rupture strength, but should not occupy a significant amount of cross-sectional area in the artery in order to minimize interference with normal fluid flow through the parent vessel. Less than five percent area occupation is desired. The thickness of the film is selected to achieve a desired frangibility at a minimum differential pressure within an acute time period to minimize ischemia downstream of the perforator vessel. In some constructions, the acute time period is preferably within a period of less than ten minutes, more preferably less than five minutes, in a majority of patients under typical conditions, that is, not including hypothermic or artificially depressed blood pressure conditions. The rupture strength should be adjusted so that the film is strong enough to survive delivery and placement within the target artery, but weak enough to rupture in the presence of the persistent, net-positive differential pressure across the ostium of small branching vessels. Desirable rupture strengths are expected to be in the range of 1 to 50 mmHg differential pressure.

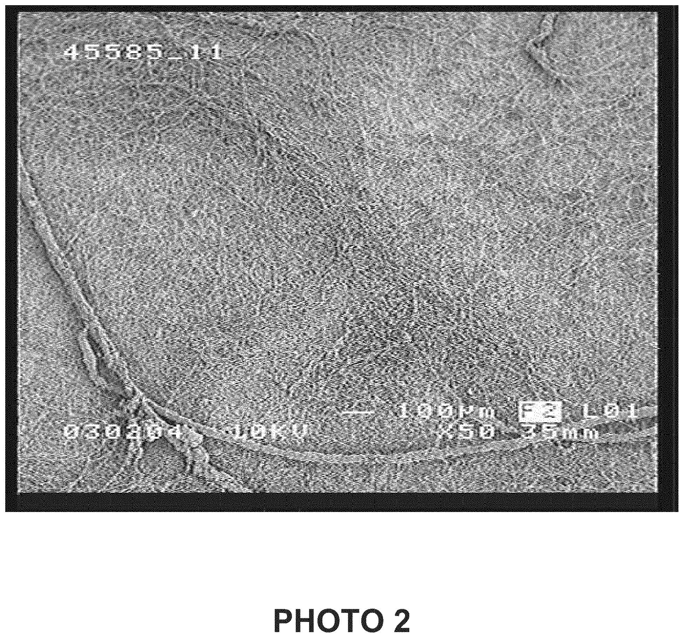

An alternative tubular device 30, FIG. 2, according to the parent invention has struts 32 which are similar to struts 12, FIG. 1, and define relatively large openings 33, FIG. 2. Device 30 further includes frangible material 34 which is formed from very thin fibers 35 in this construction that establish a porous mesh or matte outer layer. Frangible material 34 has a density sufficient to disrupt normal fluid flow at neck N to create stasis within aneurysm A to enable thrombi to form therein, yet a sufficient number of the fibers 35 part or separate to form opening 36 at the ostium of perforator vessel P when a threshold pressure differential is exceeded to enable blood to flow as illustrated by arrows 40 and 41.

In a preferred construction, these fibers 35 are applied via "electro-spinning", where a liquefied polymer such as polyvinylidene fluoride (PVDF) exiting a dispenser tip has a voltage applied to it, producing a very fine strand having an average strand thickness or diameter of one nanometer up to about ten microns. A number of controls over the construction of the fiber layer can be manipulated, such as the thickness of individual strands, the total number of strands applied, the angle at which the strand lays on the tubular scaffold, and the angles between strands which cross each other. Various electro-spinning techniques can be utilized, such as those described by Norton in U.S. Patent No. 2,048,651. Other electro-spinning techniques are described by Cooley in U.S. Patent No. 692,631, by Morton in U.S. Patent No. 705,691, and by Formhals in U.S. Patent Nos. 1,975,504 and 2,349,950 for example. The resulting characteristics of the fiber layer as manufactured, before implantation, include percentage area covered, average pore or opening size, total wall thickness, and hydraulic permeability, which provides a gross measurement of the volumetric flow rate of a certain liquid across the layer, in this case blood. In some constructions, the overall layer thickness of material 34 is about 10 microns to about 500 microns, more preferably 30 microns to 200 microns. The average opening diameter between fibers, as measured from scanning electron microscope images along a plane substantially parallel to the surface of material 35, is preferably at least 10 microns before implantation in a patient. Average openings of about 10 microns permit a small quantity of whole blood, including red blood cells, to pass through the sidewalls of device 30 to provide some nourishment to surrounding tissues, while initially providing a substantial barrier to flow through material 34. As one or more fibers rupture in the presence of sufficient differential pressure such as at the ostium of the perforator vessel P, opening 36 is preferably formed to be from 50 to 500 microns, more typically 100 to 300 microns in diameter.

One construction of device 30 is shown in PHOTOS 1-4 as scanning electron microscope images of successively smaller portions of the electro-spun fibers of device 30 at increasing magnifications of X15, X50, X200 and X2000, respectively. The left-hand side of PHOTO 1 shows fibers removed to expose the metallic struts which underlie and support the fibers, the struts defining large openings greater than one mm in this construction. A horizontal white bar illustrates a length of one mm to provide an indication of scale.

PHOTO 2 is an enlargement of the outer fiber mat layer approximately in the center of PHOTO 1. A short horizontal white bar shows a length of 100 microns. PHOTO 3 is a further enlargement showing a longer white bar also having a length of 100 microns and revealing the three-dimensional nature of the fiber mat. PHOTO 4 clearly shows the porosity of the fiber mat, with a horizontal white bar of 10 microns for scale.

The mechanism by which a sufficient number of these fibers "part" or separate in the presence of sufficient differential pressure is primarily that individual fibers will break, that is, rupture, in the localized areas of higher fluid flow. In alternate constructions, a mixture of biologically durable and degradable materials are utilized for the fibers. In regions of the fiber mesh that cover the ostium of a branching vessel, the local differential pressure is net positive and causes a persistent flow through the wall thickness of the layer. These broken fibers in the region of the layer covering the ostium of a branching vessel serve to increase the blood flow to that branching vessel preferentially compared to the region covering the aneurysm neck. The controllable factors in the construction of the frangible fiber layer 34, FIG. 2, should be adjusted such that the fibers 35 break in areas with differential pressure preselected to be a threshold rupture pressure between 1 and 50 mmHg. The thickness of the fiber layer is determined by its rupture strength, but should not occupy a significant amount of cross-sectional area in the artery. Less than five percent area occupation is desired. In some constructions, a sufficient number of fibers break or erode within an acute time period, to minimize ischemia downstream of the perforator vessel, that is preferably within a period of less than ten minutes, more preferably less than five minutes, in a majority of patients under typical conditions, that is, not including hypothermic or artificially depressed blood pressure conditions.

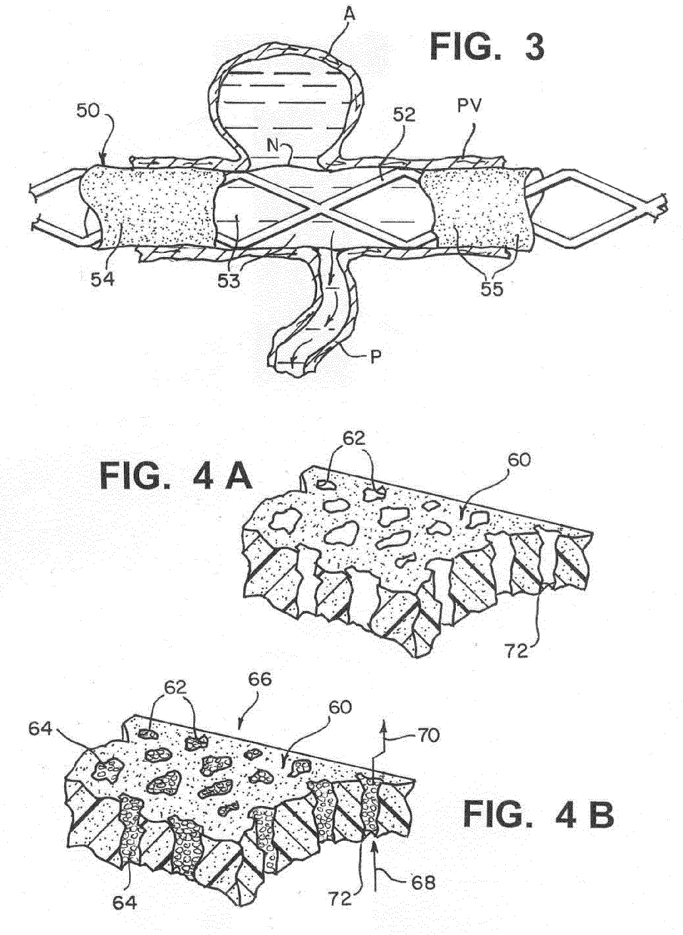

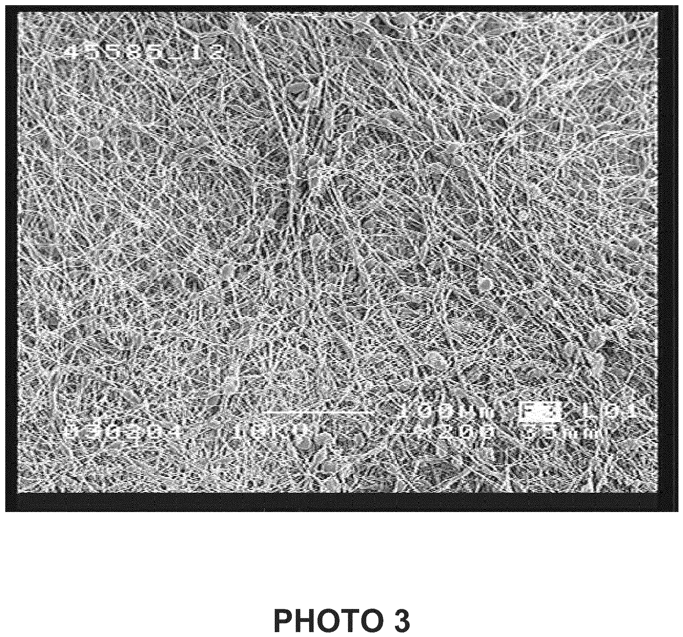

Tubular device 50, FIG. 3, is yet another embodiment of the parent invention constructed with struts 52 arranged as a scaffold to define open areas or cells 53. This scaffold 52 can be either self-expanding or balloon expanded, made by any of several typical fabrication methods. The scaffold 52 is then covered according to one technique according to the present invention with a layer 54 that has very fine pores 55 and allows a limited amount of flow across its wall thickness in the presence of a net positive differential pressure. This layer 54 can be constructed by many methods, for example foaming, lyophilization, gaseous extraction, etching, firing, or deposition. A presently preferred method is described below in relation to FIGS. 5-7. The material of layer 54 can be any biocompatible material that is subject to erosion due to fluid flow and/or erosion due to bioabsorption including consumption by live cells. In the preferred embodiment, polycaprolactone (PCL) is deposited in a somewhat sparse matrix such that it is porous as a bulk material. Other potential materials include polylactic acid (PLA), polyglycolic acid (PGA), polysaccharides, colloidal compounds, and some lipid products.

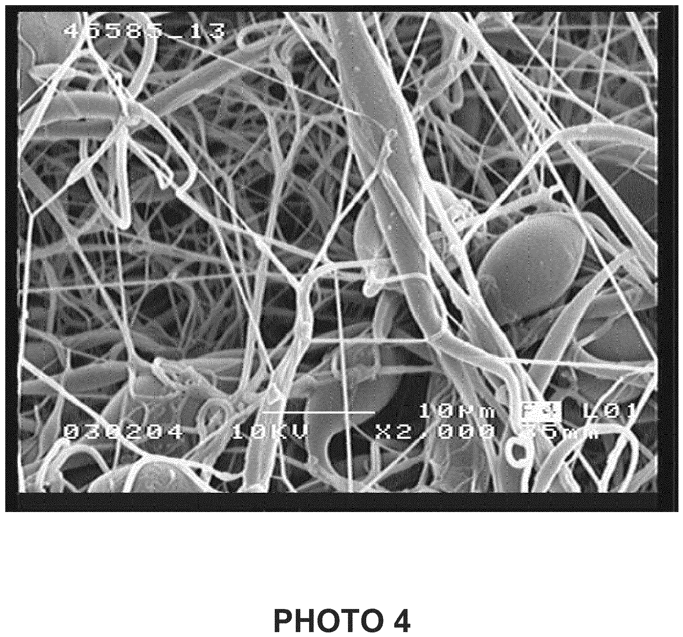

In an alternate configuration as shown in FIGS. 4A and 4B, a structure 60 of a durable, non-erodible, non-bioabsorbable material is first constructed. This flexible, elastic structure, such as a solidified urethane foam or expanded polytetrafluoroethylene (PTFE), has relatively large pores 62 so that structure 60, by itself, covers too little of the open area, has too large an average pore size, and has a hydraulic permeability that is too great to sufficiently impede or restrict flow into an aneurysm. In other words, structure 60, which may be reinforced with metal struts, establishes a maximum porosity for a device according to the present invention. Although pores 62 are shown in cross-section with relatively straight passages, such as passage 72, for simplicity of illustration, in many constructions the passages are more complex and convoluted. Pores 62 are preferably formed to be from 50 to 500 microns in average diameter, more typically 100 to 300 microns in average diameter, as measured from scanning electron microscope images along a plane substantially parallel to the surface of structure material 60.

After fabricating the structure 60, a second substance 64 that is erodible is interstitially combined with the structure 60 to form a device 66, FIG. 4B. The second material 64, such as PCL or other materials listed above, preferably is deposited as particles or a microporous foam such that the material 64 has a desired level of porosity itself, that is, it is not an impermeable bulk material. In certain constructions, material 64 defines openings having an average diameter of preferably at least 10 microns before implantation in a patient. Average openings of about 10 microns permit a small quantity of whole blood, including red blood cells, to pass through the sidewalls of device 66, as indicated by internal flow arrow 68 entering into passage 72 and external flow arrow 70 emerging from passage 72, to provide some nourishment to surrounding tissues, while initially providing a substantial barrier to flow through device 66. In the areas of net positive differential pressure, over the ostia of branching vessels, the persistent, penetrating flow through the wall of the combined layer will cause the second material 64 to respond by preferentially eroding, typically including biodegrading, more rapidly in one or more pores 62. The first purpose of the structure material 60 is to impose an upper limit on the increase in porosity, and therefore flow, to that of the structure 60 itself after all of the second material 64 has been removed. Its second purpose is to intensify the erosion, typically including biodegradation, of the second material 64 by concentrating the differential pressure provided by the branching vessel into a smaller porous area. This will improve the preferential nature by which the combined layer of device 66 will erode above branching vessels more quickly than in the general body of the device, including above an aneurysm neck.

A presently preferred method for fabricating a frangible layer utilizes spray phase separation established by at least two spray devices. As illustrated schematically in FIG. 5, a spray system 100 includes a spray apparatus 101 with a first spray device 102 having an adjustable nozzle 104, a nozzle opening adjustment knob 106, and a flow control knob 108. A second spray device 110 has an adjustable nozzle 112, a nozzle opening adjustment knob 114, and a flow control knob 116. One full turn or rotation of flow control knob 108 or knob 116 is referred to as a revolution or "rev". Spray devices 102 and 110 are mounted on a bracket 120 which, in some constructions, includes a carriage for spray apparatus 101 movable in a direction such as indicated by arrow 122. The openings of nozzles 104 and 112 are adjusted to create spray patterns 130 and 132, respectively, which overlap at collection region 134 on a cylindrical mandrel 140. In certain constructions, mandrel 140 is moved in a direction such as represented by arrow 142. Nozzles 104 and 112 are positioned at a pre-selected distance PD and WD, respectively, from collection region 134. Suitable spray devices include AOM Asturo 878 WB Mini HVLP spray guns available from Asturo Spray Equipment, Rio Rancho, New Mexico.

Presently preferred ranges of settings for spray system 100 include those shown in Table I:

| **Parameter** | **Low Setting** | **High Setting** |
|---|---|---|
| Relative Translation Speed | 11 cm/sec | 33 cm/sec |
| Distance | 25 cm | 33 cm |
| Polymer Nozzle Diameter | 0.8mm | 1.2mm |
| Non-Solvent Nozzle Diameter | 0.8mm | 1.2mm |
| Polymer Flow | 1 rev | 2 rev |
| Non-Solvent Flow | 0.25 rev | 1 rev |

The parameters shown in Table I are presently preferred for delivering a first polymer solution of polycaprolactone and polyurethane, preferably in a blend ratio of 80:20 to 50:50, through first spray device 102 and for delivering water as the non-solvent through second spray device 110. In certain constructions, mandrel 140 is rotated about its longitudinal axis at speeds of 600 revolutions per minute to form a tubular porous membrane. This process preferably is conducted at standard temperature and pressure, with relative humidity preferably held to less than twenty percent.

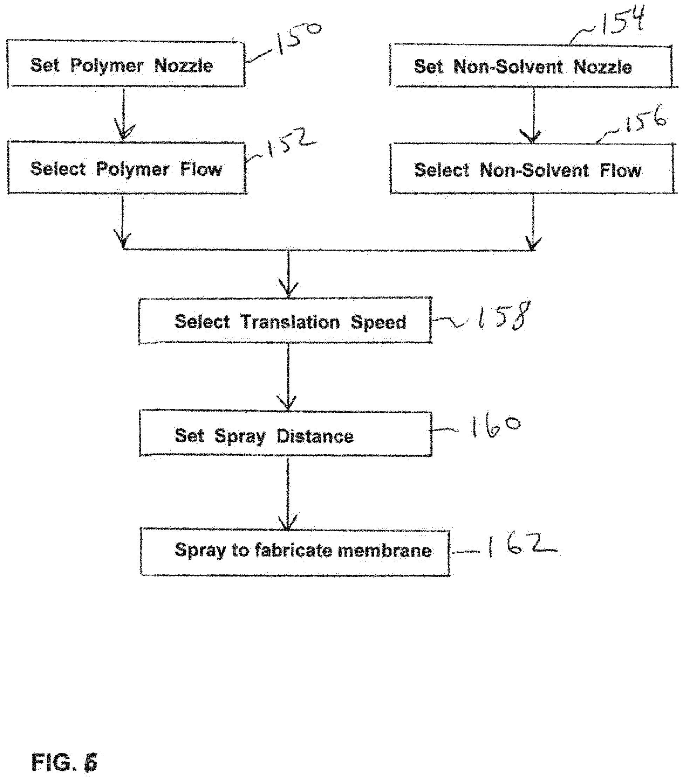

FIG. 6 is a flow chart outlining steps for operating system 100, FIG. 5. The effective opening diameter of polymer nozzle 104 is set, step 150, and a flow rate for the polymer solution is selected, step 152. Similarly, the effective opening diameter of non-solvent nozzle 112 is set, step 154, and a flow rate for the non-solvent solution is selected, step 152. The relative translation speed between the spray apparatus 101 and the mandrel 120 is selected, step 158, and spray distance PD and WD is set, step 160. System 100 is then operated at the pre-selected parameters to fabricate a porous membrane, step 162.

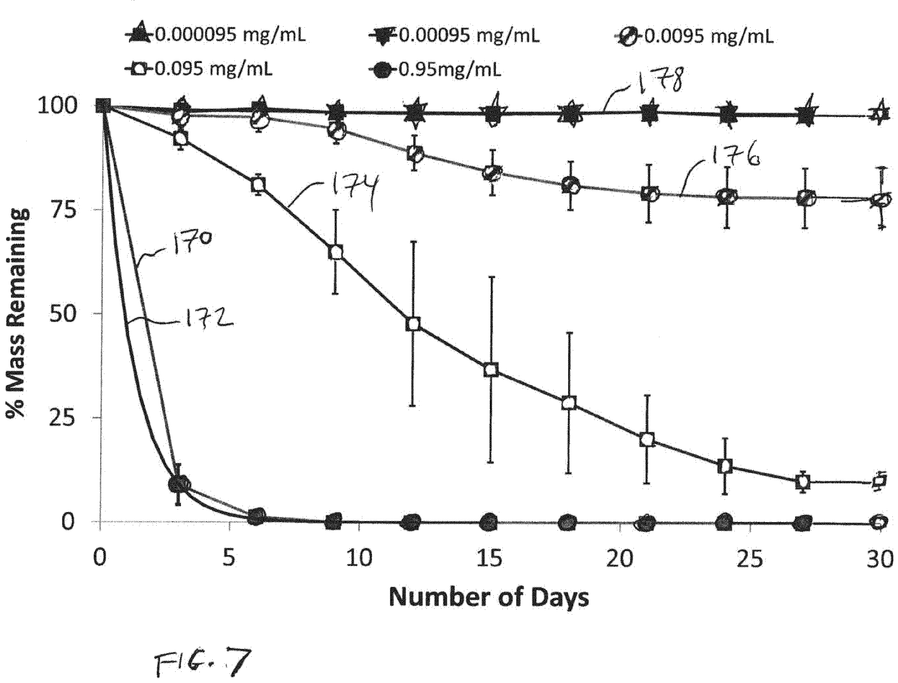

FIG. 7 is a graph showing degradation of membranes formed according to the present invention with pure PCL in varying lipase concentrations. Percentage mass remaining is shown in the Y-axis and the number of days at which measurements were taken is shown on the X-axis. The immersion solution was changed every three days, and results are shown for three samples at each concentration. Curve 170 shows straight-line segments connecting points over time, with exponential function fit curve 172, for lipase concentrations of 0.95 mg/mL. Similarly, curves 174 and 176 show mass remaining over time for lipase concentrations of 0.095 mg/mL and 0.0095 mg/mL lipase, respectively. Curves 174 and 176 are best approximated with cubic functions instead of exponential functions. Also shown is a single overlapping curve 178 for lipase concentrations 0.00095 and 0.000095 mg/mL lipase, respectively.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. A method of fabricating a frangible material (14) for an occlusive device suitable for endovascular treatment of an aneurysm in a region of a parent vessel in a patient, comprising:
selecting a first spray device (102) having a first adjustable nozzle opening and a first adjustable flow control;
selecting a second spray device (110) having a second adjustable nozzle opening and a second adjustable flow control;
selecting for the first flow control a position of one unit to two units;
selecting for the second flow control a position of 0.25 units to one unit;
arranging the first and second spray devices (102, 110) to deliver droplets of a first liquid including at least one biocompatible polymer through the first spray device and to deliver droplets of a second liquid including a non-solvent for the polymer through the second spray device in an overlapping spray pattern on a substrate at a distance of 25 cm to 35 cm;
selecting a relative translation speed of 11 cm/sec to 33 cm/sec between (i) the first and second spray devices and (ii) the substrate; and
spraying the polymer and the non-solvent onto the substrate to cause the biocompatible polymer to disassociate from solution to form the frangible material (14) as a porous membrane.

2. The method of claim 1 wherein the substrate is a mandrel (140).

3. The method of claim 1 wherein the at least one polymer is polycaprolactone.

4. The method of claim 3 wherein the first liquid further includes polyurethane.

5. A method according to claim 1, further comprising the steps of
setting the first adjustable nozzle opening to a diameter between 0.8 mm to 1.2 mm and
setting the second adjustable nozzle opening to a diameter between 0.8 mm to 1.2 mm
prior to selecting for the first flow control position and second flow control position, wherein the first liquid further includes at least one biodegradable polymer .

6. The method of claim 1 or claim 5 wherein the substrate is a substantially cylindrical mandrel (140).

7. The method of claim 6 when dependent upon claim 5 wherein the at least one biodegradable polymer is polycaprolactone.

8. The method of claim 7 wherein the at least one biocompatible polymer is polyurethane.

9. The method of claim 1 or claim 5 wherein the frangible material (14), upon implantation in the parent vessel, initially provides a substantial barrier to flow through the frangible material and is capable of localized eroding, in the presence of a pressure differential arising at an ostium of a perforator vessel communicating with the parent vessel, within an acute time period to minimize ischemia downstream of the perforator vessel.

10. The method of claim 9 further including, prior to implantation, placing the frangible material (14) over a structure having a fixed porosity and having dimensions suitable for insertion into vasculature of the patient to reach the region of the aneurysm in the parent vessel.

11. The method of claim 10 wherein the structure includes metallic struts.

12. The method of claim 9 wherein at least a substantial amount of the surface area of the frangible material (14) defines openings at least 10 microns in diameter prior to implantation in the patient.

13. The method of claim 9 wherein the frangible material (14) has a thickness ranging between 10 microns to 500 microns prior to implantation in the patient.

14. The method of claim 9 wherein the frangible material (14) is capable of responding to a pressure differential equivalent to 0.1 to 6.7 kPa (one to fifty mm Hg).

15. The method of claim 9 wherein the acute time period is less than ten minutes.

16. The method of claim 5 wherein the second liquid includes water as the non-solvent.

17. An occlusive device formed by the method of claim 10.

## Patentansprüche

1. Verfahren zur Herstellung eines fragilen Materials (14) für eine Okklusionsvorrichtung, die zur endovaskulären Behandlung eines Aneurysmas in einer Region eines Stammgefäßes in einem Patienten geeignet ist, umfassend:
Auswählen einer ersten Sprühvorrichtung (102) mit einer ersten justierbaren Düsenöffnung und einer ersten justierbaren Flusssteuerung;
Auswählen einer zweiten Sprühvorrichtung (110) mit einer zweiten justierbaren Düsenöffnung und einer zweiten justierbaren Flusssteuerung;
Auswählen einer Position von einer Einheit bis zwei Einheiten für die erste Flusssteuerung;
Auswählen einer Position von 0,25 Einheiten bis einer Einheit für die zweite Flusssteuerung;
Anordnen der ersten und zweiten Sprühvorrichtung (102, 110), um Tröpfchen einer ersten Flüssigkeit, die mindestens ein biokompatibles Polymer einschließt, durch die erste Sprühvorrichtung abzugeben und Tröpfchen einer zweiten Flüssigkeit, die ein Nichtlösungsmittel für das Polymer einschließt, durch die zweite Sprühvorrichtung in einem überlappenden Sprühmuster auf ein Substrat in einem Abstand von 25 cm bis 35 cm abzugeben;
Auswählen einer relativen Translationsgeschwindigkeit von 11 cm/s bis 33 cm/s zwischen (i) der ersten und zweiten Sprühvorrichtung und (ii) dem Substrat; und
Sprühen des Polymers und des Nichtlösungsmittels auf das Substrat, um herbeizuführen, dass sich das biokompatible Polymer aus der Lösung absondert, um das fragile Material (14) als poröse Membran zu bilden.

2. Verfahren nach Anspruch 1, wobei das Substrat ein Mandrin (140) ist.

3. Verfahren nach Anspruch 1, wobei das mindestens eine Polymer Polycaprolacton ist.

4. Verfahren nach Anspruch 3, wobei die erste Flüssigkeit ferner Polyurethan einschließt.

5. Verfahren nach Anspruch 1, ferner umfassend die folgenden Schritte:
Einstellen der ersten justierbaren Düsenöffnung auf einen Durchmesser zwischen 0,8 mm und 1,2 mm und
Einstellen der zweiten justierbaren Düsenöffnung auf einen Durchmesser zwischen 0,8 mm und 1,2 mm vor dem Auswählen der ersten Flusssteuerungsposition und der zweiten Flusssteuerungsposition, wobei die erste Flüssigkeit ferner mindestens ein biologisch abbaubares Polymer einschließt.

6. Verfahren nach Anspruch 1 oder Anspruch 5, wobei das Substrat ein im Wesentlichen zylindrischer Mandrin (140) ist.

7. Verfahren nach Anspruch 6 in Abhängigkeit von Anspruch 5, wobei das mindestens eine biologisch abbaubare Polymer Polycaprolacton ist.

8. Verfahren nach Anspruch 7, wobei das mindestens eine biokompatible Polymer Polyurethan ist.

9. Verfahren nach Anspruch 1 oder Anspruch 5, wobei das fragile Material (14) beim Implantieren in das Stammgefäß anfangs eine erhebliche Barriere für das Fließen durch das fragile Material hindurch bereitstellt, und in der Lage ist, in Anwesenheit einer Druckdifferenz, die an einem Ostium eines Perforatorgefäßes entsteht, das mit dem Stammgefäß in Verbindung steht, innerhalb eines akuten Zeitraums örtlich begrenzt zu erodieren, um Ischämie nachgeordnet zu dem Perforatorgefäß zu minimieren.

10. Verfahren nach Anspruch 9, ferner einschließend Platzieren des fragilen Materials (14) über einer Struktur mit fester Porosität und mit Abmessungen, die für das Einführen in das Gefäßsystem des Patienten geeignet sind, vor der Implantierung, um die Region des Aneurysmas in dem Stammgefäß zu erreichen.

11. Verfahren nach Anspruch 10, wobei die Struktur metallische Streben einschließt.

12. Verfahren nach Anspruch 9, wobei mindestens ein wesentlicher Betrag der Oberfläche des fragilen Materials (14) Öffnungen mit einem Durchmesser von mindestens 10 Mikrometer vor der Implantierung in den Patienten definiert.

13. Verfahren nach Anspruch 9, wobei das fragile Material (14) vor der Implantierung in den Patienten eine Dicke im Bereich von 10 Mikrometern bis 500 Mikrometern aufweist.

14. Verfahren nach Anspruch 9, wobei das fragile Material (14) in der Lage ist, auf eine Druckdifferenz zu reagieren, die 0,1 bis 6,7 kPa (ein bis fünfzig mm Hg) entspricht.

15. Verfahren nach Anspruch 9, wobei der akute Zeitraum weniger als zehn Minuten beträgt.

16. Verfahren nach Anspruch 5, wobei die zweite Flüssigkeit Wasser als Nichtlösungsmittel einschließt.

17. Okklusionsvorrichtung, die nach dem Verfahren von Anspruch 10 hergestellt ist.

## Revendications

1. Procédé de fabrication d'un matériau frangible (14) pour un dispositif d'occlusion approprié pour un traitement endovasculaire d'un anévrisme dans une région d'un vaisseau parent dans un patient, comprenant les étapes suivantes:
sélectionner un premier dispositif de pulvérisation (102) comportant une première ouverture de buse réglable et une première commande d'écoulement réglable;
sélectionner un second dispositif de pulvérisation (110) comportant une seconde ouverture de buse réglable et une seconde commande d'écoulement réglable;
sélectionner pour la première commande d'écoulement une position d'une unité à deux unités;
sélectionner pour la seconde commande d'écoulement une position de 0,25 unité à une unité;
agencer les premier et second dispositifs de pulvérisation (102, 110) de manière à distribuer des gouttelettes d'un premier liquide comprenant au moins un polymère biocompatible à travers le premier dispositif de pulvérisation et à distribuer des gouttelettes d'un second liquide comprenant un non-solvant pour le polymère à travers le second dispositif de pulvérisation dans un motif de pulvérisation à chevauchement sur un substrat à une distance comprise entre 25 cm et 35 cm;
sélectionner une vitesse de transition relative de 11 cm/sec à 33 cm/sec entre (i) les premier et second dispositifs de pulvérisation et (ii) le substrat; et
pulvériser le polymère et le non-solvant sur le substrat afin d'amener le polymère biocompatible à se dissocier de la solution de manière à former le matériau frangible (14) comme une membrane poreuse.

2. Procédé selon la revendication 1, dans lequel le substrat est un mandrin (140).

3. Procédé selon la revendication 1, dans lequel ledit au moins un polymère est le polycaprolactone.

4. Procédé selon la revendication 3, dans lequel le premier liquide comprend en outre du polyuréthane.

5. Procédé selon la revendication 1, comprenant en outre les étapes suivantes:
régler la première ouverture de buse réglable à un diamètre compris entre 0,8 mm à 1,2 mm; et
régler la seconde ouverture de buse réglable à un diamètre compris entre 0,8 mm à 1,2 mm avant de sélectionner la première position de commande d'écoulement et la seconde position de commande d'écoulement,
dans lequel le premier liquide comprend en outre au moins un polymère biodégradable.

6. Procédé selon la revendication 1 ou la revendication 5, dans lequel le substrat est un mandrin sensiblement cylindrique (140).

7. Procédé selon la revendication 6 lorsqu'elle dépend de la revendication 5, dans lequel ledit au moins un polymère biodégradable est le polycaprolactone.

8. Procédé selon la revendication 7, dans lequel ledit au moins un polymère biocompatible est le polyuréthane.

9. Procédé selon la revendication 1 ou la revendication 5, dans lequel le matériau frangible (14), lors de son implantation dans le vaisseau parent, fournit initialement une barrière substantielle à l'écoulement à travers le matériau frangible et est capable d'une érosion localisée, en présence d'un différentiel de pression survenant à un ostium d'un vaisseau perforant qui communique avec le vaisseau parent, à l'intérieur d'une période de temps critique afin de minimise une ischémie en aval du vaisseau perforant.

10. Procédé selon la revendication 9, comprenant en outre, avant l'implantation, le placement du matériau frangible (14) sur une structure présentant une porosité fixe et présentant des dimensions appropriées pour une insertion dans le système vasculaire du patient pour atteindre la région de l'anévrisme dans le vaisseau parent.

11. Procédé selon la revendication 10, dans lequel la structure comprend des entretoises métalliques.

12. Procédé selon la revendication 9, dans lequel au moins une quantité substantielle de l'aire de surface du matériau frangible (14) définit des ouvertures d'au moins 10 microns de diamètre avant l'implantation dans le patient.

13. Procédé selon la revendication 9, dans lequel le matériau frangible (14) présente une épaisseur comprise dans la gamme de 10 microns à 500 microns avant l'implantation dans le patient.

14. Procédé selon la revendication 9, dans lequel le matériau frangible (14) est capable de réagir à un différentiel de pression équivalent à 0,1 kPa à 6,7 kPa (un à cinquante mm Hg).

15. Procédé selon la revendication 9, dans lequel la période de temps critique est inférieure à dix minutes.

16. Procédé selon la revendication 5, dans lequel le second liquide comprend l'eau comme non-solvant.

17. Dispositif d'occlusion formé par le procédé selon la revendication 10.
